# FASCICULE DE BREVET EUROPEEN

(11) **EP 3 630 051 B1**
(45) Date de publication et mention de la délivrance du brevet: **17.11.2021**
(21) Numéro de dépôt: 18726977.4
(22) Date de dépôt: 23.05.2018
(51) Int. Cl.: A61K 8/34, A61K 8/44, A61K 8/60, A61K 8/73, A61Q 19/00, A61K 8/97, A61L 15/00, A61K 8/06

(54) **COMPOSITION POUR LE REMODELAGE DES CICATRICES**
ZUSAMMENSETZUNG FÜR DIE REMODELLIERUNG VON NARBEN
COMPOSITION FOR REMODELING SCARS

(30) Priorité: 24.05.2017 FR 1754598
(43) Date de publication de la demande: 08.04.2020
(73) Titulaire: Pierre Fabre Dermo-Cosmétique, 92100 Boulogne-Billancourt (FR)
(72) Inventeur: BIDAN, Catherine, 31860 Labarthe Sur Leze (FR); RATTIER, Sandy, 31400 Toulouse (FR)
(74) Mandataire: Ipside
(86) Numéro de dépôt international: PCT/EP2018/063472
(87) Numéro de publication internationale: WO 2018/215522

(56) Documents cités:
- WO-A1-2014/167039
- DATABASE GNPD [Online] MINTEL; 1 février 2017 (2017-02-01), "Ultra Repairing Cream", XP002775619, Database accession no. 4609609
- DATABASE GNPD [Online] MINTEL; 1 octobre 2015 (2015-10-01), "Epitheliale A.H Anti-Marks", XP002775620, Database accession no. 3490843

## Description

La présente invention s'inscrit dans le domaine du remodelage des cicatrices, après refermeture de la lésion qui en est la source.

Plus particulièrement, la présente invention concerne l'utilisation d'une composition pour le traitement d'une cicatrice par voie topique, en particulier en tant qu'adjuvant de massage.

La cicatrisation est un processus physiologique de réparation tissulaire, intervenant après qu'une plaie ou lésion, c'est-à-dire une destruction du revêtement cutané, se soit formée consécutivement à l'action d'un agent mécanique externe ou à une agression telle qu'un traumatisme direct, une brûlure, une blessure, une intervention chirurgicale, etc. La cicatrisation permet de rétablir la peau dans son intégralité. Ce processus aboutit cependant généralement à la formation d'une cicatrice visible, qui forme une marque sur la peau.

Les procédés d'aide à la cicatrisation s'inscrivent généralement dans le cadre de trois phases successives. Dans une première phase d'évolution de la lésion cutanée, dite phase vasculaire ou inflammatoire, l'objectif des traitements est d'arrêter le saignement et de nettoyer la plaie. Cette phase se déroule dans les premières heures après la formation de la lésion. Elle dure généralement de quelques heures à quelques jours. Dans une deuxième phase, dite de réparation tissulaire ou proliférative, les traitements visent à faciliter la refermeture de la plaie et le comblement de la substance manquante, par régénération des tissus. Cette deuxième phase dure typiquement de quelques jours à quelques semaines, plus précisément de deux ou trois jours à deux ou trois semaines, selon l'importance de la lésion initiale. A l'issue de cette phase, la réépidermisation de la lésion initiale est complète, et une cicatrice reste généralement visible sur la peau. Enfin, dans une troisième phase, dite phase de maturation ou de remodelage, l'objectif des traitements est de remodeler la cicatrice qui s'est formée au cours de la deuxième phase, et de lui donner un aspect le plus esthétique possible. Cette phase peut durer de deux mois à deux ans. C'est pendant cette période que l'évolution de la cicatrice se déroule réellement. Les fibres de collagène se réorganisent et se densifient.

La présente invention s'inscrit plus particulièrement dans le cadre des procédés d'aide à la cicatrisation mis en œuvre lors de la troisième phase de la cicatrisation, c'est-à-dire lors de la phase de remodelage des cicatrices, après que la lésion initiale se soit complètement refermée, lorsque la réépidermisation de la lésion initiale est complète.

Au cours de cette phase de remodelage, les fibres qui se forment peuvent s'accrocher aux muscles ou aux tissus sous la peau, et créer ainsi ce qu'il est commun d'appeler des adhérences. Ces dernières peuvent être sources de problèmes et provoquer, notamment, douleur et/ou raideur.

Afin de remédier à ce problème, et de favoriser plus généralement l'évolution positive de la phase de remodelage des cicatrices, et en particulier éviter une évolution en cicatrice hypertrophique inesthétique, il est préconisé de procéder à des massages, notamment selon la technique dite du palper-rouler, des cicatrices après la réépidermisation complète de la lésion qui en est à l'origine, et ce pendant une durée d'environ deux mois jusqu'à environ deux ans. Cette action mécanique exercée sur les cicatrices permet de les assouplir, d'améliorer l'élasticité de la peau à cet endroit et de rendre leur liberté aux tissus, en cassant les adhérences et en rétablissant les plans de glissement au plus proche de la physiologie. Les massages améliorent également la vascularisation tissulaire et ils diminuent le prurit. Des huiles de massage, des gels à base de silicone ou des pommades hydratantes peuvent être utilisés au cours de tels massages. Si ces produits facilitent la glisse des doigts du masseur sur la surface cutanée, ils n'ont cependant quasiment aucune efficacité en eux-mêmes sur l'apparence de la cicatrice. Les gels à base de silicone ont en outre un effet occlusif, indésirable ; ils peuvent par ailleurs être allergisants.

La présente invention vise à proposer une composition cosmétique et/ou dermatologique qui, mise en œuvre lors de la phase de remodelage d'une cicatrice, c'est-à-dire après la réépidermisation complète de la lésion initiale, lorsque la lésion est complètement refermée, permette par une application par voie topique, en complément des techniques de massage existantes, de favoriser le remodelage de la cicatrice et d'améliorer de manière efficace son aspect esthétique, diminuant par là-même le risque que l'individu concerné ne conserve définitivement une marque cicatricielle.

Un objectif supplémentaire de l'invention est que cette composition contienne sensiblement uniquement des ingrédients naturels et/ou d'origine naturelle, issus de ressources renouvelables, par opposition aux ressources fossiles, ces ingrédients pouvant de préférence être obtenus par des procédés respectueux de l'environnement.

A cet effet, il est proposé selon la présente invention une composition cosmétique et/ou dermatologique pour son utilisation pour le traitement, plus particulièrement par remodelage cellulaire, des cicatrices par voie topique, telle que définie dans la revendication 1. La composition selon l'invention est destinée à être mise en œuvre en tant qu'adjuvant de massage.

Cette composition, applicable par voie topique, c'est-à-dire de forme adaptée pour une application par voie topique, se présente sous forme d'une microémulsion comprenant une phase lipophile et une phase aqueuse, sous forme de gel. Elle contient :
- un composé lipophile,
- un agent cicatrisant,
- un agent humectant,
- un agent émulsionnant, apte à former une microémulsion, contenant au moins un composé choisi parmi :
   o les esters d'acides gras et de polyglycérine
   o et les mélanges d'un ester de sucre, d'un polyol et d'un composé lipophile choisi parmi les triglycérides ou l'un quelconque de leurs mélanges
- et de l'eau dans une quantité totale comprise entre 1 et 10 % en poids par rapport au poids total de la composition.

La quantité totale en composé(s) lipophile(s) dans la composition est en outre de préférence supérieure ou égale à 40 % en poids par rapport au poids total de la composition. Une telle caractéristique, associée à la forme particulière de microémulsion et à la faible teneur en eau de la composition, confère avantageusement à la composition selon l'invention des caractéristiques organoleptiques très proches de celles des huiles, tout en étant plus visqueuse qu'une huile, ce qui favorise son utilisation pour les massages prolongés de la surface cutanée.

Dans le terme cicatrices, on inclut selon la présente invention les cicatrices à proprement parler, c'est-à-dire les zones de tissus fibreux remplaçant dans le derme les tissus normaux après une lésion cutanée, mais également les vergetures, qui sont définies comme de fines raies cutanées d'aspect cicatriciel, dues à la distension ou à la rupture de fibres élastiques du derme, et plus généralement toutes zones de peau fragilisée après réépidermisation, présentant un risque de marques cicatricielles. Dans tous les cas, on exclut du terme cicatrice, au sens de la présente invention, toute plaie ou lésion non encore complètement refermée.

On entend dans la présente description, par microémulsion, de manière classique en elle-même, une composition comprenant une phase lipophile, dite phase grasse, et une phase aqueuse, l'une de ces phases étant dispersée en fines gouttelettes dans l'autre de ces phases, dans laquelle la taille des gouttelettes d'une phase dispersées dans l'autre phase est typiquement de l'ordre de quelques dizaines de nanomètres. Les microémulsions se caractérisent notamment par un état thermodynamiquement stable, et un aspect translucide. Il entre dans les compétences de l'homme du métier de choisir l'agent émulsionnant adéquat pour former une microémulsion conformément à l'invention.

Par agent cicatrisant, on entend dans la présente description un agent favorisant la réparation de l'épiderme.

La composition selon l'invention se présente à l'état initial sous forme d'un gel. On peut alors la qualifier de gel huileux, ou d'huile gélifiée, en raison de la faible proportion en eau qu'elle contient, notamment en regard d'une teneur en composé(s) lipophiles(s) élevée, et de préférence supérieure ou égale à 40 % en poids par rapport au poids total de la composition. La composition selon l'invention présente ainsi de préférence, à l'état initial, une viscosité dynamique, mesurée à 22 °C, supérieure ou égale à 8 000 mPa.s. Une telle viscosité, et une telle texture de gel, peuvent être obtenues par un choix adéquat de l'agent émulsionnant, parmi les agents émulsionnants présentant également des propriétés gélifiantes, ou encore en adjoignant à l'agent émulsionnant un agent à propriétés gélifiantes.

La composition selon l'invention présente de préférence une viscosité dynamique à l'état initial, mesurée à 22 °C, comprise entre 10000 et 22000 mPa.s, notamment comprise entre 10000 et 16000 mPa.s ou comprise entre 16000 et 22000 mPa.s, en fonction de l'agent émulsionnant, et, le cas échéant, de l'agent gélifiant, mis en œuvre. Une telle viscosité est particulièrement adaptée à une application sur la peau par massage, notamment car la composition ne s'écoule alors pas après son application sur la surface cutanée.

La composition selon l'invention peut consister en une microémulsion du type eau-dans-huile, c'est-à-dire dans laquelle la phase aqueuse se trouve dispersée sous forme de fines gouttelettes dans la phase lipophile. Préférentiellement, elle se présente sous forme de microémulsion de type huile-dans-eau, c'est-à-dire dans laquelle la phase lipophile se trouve dispersée sous forme de fines gouttelettes dans la phase aqueuse. Un tel mode de réalisation facilite le rinçage de la composition après son application sur la peau, ce qui s'avère par exemple particulièrement avantageux lorsque la zone de peau traitée se trouve à proximité des cheveux. Là encore, il entre dans les compétences de l'homme du métier de choisir l'agent émulsionnant adéquat pour obtenir le type de microémulsion souhaité.

La composition contient en outre de préférence un agent, dit agent chauffant, apte à produire de la chaleur lorsqu'il est appliqué sur la surface cutanée, notamment par massage.

La composition selon l'invention combine avantageusement les effets d'un agent cicatrisant, qui aide à lisser et aplanir la cicatrice et réduit les rougeurs, tensions et démangeaisons ; d'un agent humectant qui, par un effet d'hydratation de l'épiderme et de soulagement des démangeaisons, augmente avantageusement l'efficacité de la composition pour le remodelage des cicatrices ; et d'un composé lipophile, à l'effet duquel s'ajoute celui de la texture en gel de la composition, ces effets facilitant tous deux, lors du massage, la glisse des doigts sur la peau.

La présence supplémentaire d'un agent chauffant, qui active de manière bénéfique la microcirculation sanguine grâce à la chaleur dégagée lors de l'application par massage de la composition sur la surface cutanée, augmente plus encore l'efficacité de la composition pour le remodelage des cicatrices.

La composition selon l'invention, de par sa forme de microémulsion et sa faible teneur en eau, présente avantageusement un aspect transparent, et des caractéristiques organoleptiques très proches de celles des huiles, tout en étant plus visqueuse qu'une huile. Elle est de ce fait particulièrement adaptée pour le massage de la peau, notamment pour les massages prolongés recommandés pour le remodelage des cicatrices : elle ne s'écoule pas après son application sur la surface cutanée, elle présente un toucher huileux qui favorise le massage, et elle pénètre lentement dans la peau, par exemple bien plus lentement que les pommades proposées par le document WO 2014/167039.

La composition selon l'invention est en outre de préférence de type rhéofluidifiant, c'est-à-dire que sa viscosité diminue si la contrainte de cisaillement ou la vitesse de déformation qui lui est appliquée augmente. Ainsi, sous l'effet des mouvements exercés sur elle lors de son application par voie topique sur la cicatrice, sa viscosité diminue, elle se fluidifie et pénètre alors immédiatement dans l'épiderme. Une telle caractéristique, que l'on peut qualifier de texture évolutive, s'avère particulièrement avantageusement dans le cadre d'une application au remodelage des cicatrices. En effet, elle favorise les temps d'application longs, l'utilisateur étant naturellement incité à prolonger les mouvements nécessaires à l'application de la composition, par massage de la cicatrice, pour transformer totalement le gel et permettre son absorption dans l'épiderme. Comme il a été exposé ci-avant, le massage d'une cicatrice, qui est ainsi favorisé par les propriétés rhéologiques de la composition selon l'invention, s'avère particulièrement souhaitable lors de la phase de remodelage d'une cicatrice. Il est du ressort de l'homme du métier de déterminer quels ingrédients mettre en œuvre dans la composition selon l'invention pour obtenir une telle texture évolutive, notamment par un choix adéquat de l'agent émulsionnant.

Appliquée sur une cicatrice par voie topique, de par les effets combinés de ses constituants, la composition selon l'invention s'avère particulièrement efficace pour favoriser le remodelage de l'épiderme en complément d'un massage, et améliorer l'aspect esthétique des cicatrices, en atténuant les marques cicatricielles. Son efficacité se mesure également en termes d'apaisement immédiat et durable de la zone de peau concernée, notamment des sensations de tiraillement, ainsi que de diminution de la douleur et de la sensation de raideur au niveau de la cicatrice. Par son effet chauffant lors du massage, qui active la microcirculation sanguine, elle diminue en outre le ressenti douloureux occasionné par le massage.

Les ingrédients de la composition selon l'invention sont par ailleurs préférentiellement choisis pour que cette composition soit translucide, d'aspect agréable à la vue.

L'agent cicatrisant de la composition selon l'invention contient de préférence de l'acide hyaluronique ou un de ses sels.

L'acide hyaluronique favorise notamment l'activation et la migration cellulaire, et diminue les conséquences de l'inflammation, ce qui participe utilement au remodelage cutané. Au cours de la phase de remodelage de la cicatrice, qui conditionne son aspect esthétique futur, l'acide hyaluronique présente un effet structurant, notamment par son action sur la cohésion cellulaire et la néo-vascularisation. Il évite en outre la formation de croûtes.

L'agent cicatrisant de la composition selon l'invention contient préférentiellement une association comprenant de l'acide hyaluronique ou un de ses sels, de la L-alanyl-L-glutamine et un extrait d'avoine. Une telle association augmente de manière particulièrement importante la migration des kératinocytes au niveau de la cicatrice, par une synergie des effets de chacun de ses composants, l'extrait d'avoine présentant en particulier un effet hydratant, et le dipeptide L-alanyl-L-glutamine un effet glucotransformateur.

Cette association peut répondre à l'une ou plusieurs, de préférence à toutes, les caractéristiques suivantes :
- l'acide hyaluronique se présente sous la forme de fragments d'hyaluronate de sodium ;
- le poids moléculaire de ces fragments d'hyaluronate de sodium est compris entre 50 et 750 kDa ;
- l'extrait d'avoine est obtenu à partir de plantules d'avoine ;
- le ratio massique acide hyaluronique / extrait de plantules d'avoine / L-alanyl-L-glutamine est compris entre 2/1/3 et 2/1/5, et par exemple d'environ 2/1/4.

Une telle association et ses caractéristiques sont par exemple décrites dans le document WO 2014/167039.

La composition selon l'invention peut contenir un seul agent cicatrisant, ou un mélange de tels agents.

Des agents cicatrisants pouvant être présents dans la composition selon l'invention sont par exemple le sucralfate, le madécassoside, le panthénol, l'allantoïne, l'aloe vera ou le miel.

La quantité totale d'agent(s) cicatrisant(s) dans la composition selon l'invention est de préférence comprise entre 0,01 et 3 % en poids par rapport au poids total de la composition.

La composition selon l'invention peut en outre contenir un seul agent humectant, ou un mélange de tels agents.

Un agent humectant contenu dans la composition selon l'invention peut notamment être un polyol. Il peut notamment consister en un polyéthylène glycol, en un polypropylène glycol, tel que le dipropylène glycol, etc.

Préférentiellement, la composition selon l'invention contient de la glycérine, qui offre l'avantage de constituer à elle seule un agent humectant, émollient et hydratant physiologique, et un agent chauffant au sens de la présente invention, c'est-à-dire un agent dégageant de la chaleur lors du massage.

La glycérine permet en outre de jouer sur la transparence de la composition.

Elle présente en particulier une forte capacité de rétention en eau à la surface cutanée, et donc une forte capacité de maintien d'une hydratation de surface.

La glycérine permet également avantageusement de mettre en œuvre dans la composition selon l'invention des agents cicatrisants hydrosolubles, sans nécessiter pour autant d'incorporer d'importantes quantités d'eau dans la composition. Ceci s'avère particulièrement utile lorsque l'agent cicatrisant est une association d'acide hyaluronique ou d'un de ses sels, de L-alanyl-L-glutamine et d'un extrait d'avoine, comme décrit ci-dessus. Une telle association, qui n'est pas soluble dans les corps gras, peut être solubilisée dans la glycérine sans recours à des quantités d'eau importantes. Il est ainsi possible de préparer une composition sous forme d'un gel huileux visqueux, particulièrement bien adaptée aux massages, contenant un actif cicatrisant hydrosoluble.

La glycérine offre en outre l'avantage d'être biosourcée, et facilement disponible sous forme naturelle ou d'origine naturelle. Elle peut par exemple être extraite de l'huile de coprah ou de l'huile de palme.

La quantité totale d'agent(s) humectant(s), et plus particulièrement de glycérine, dans la composition peut notamment être comprise entre 10 et 50 % en poids par rapport au poids total de la composition, de préférence supérieure à 25 % et inférieure ou égale à 50 % en poids par rapport au poids total de la composition. Elle peut par exemple être égale à environ 30 % en poids, par rapport au poids total de la composition.

Un taux de glycérine supérieur à 25 % p/p assure notamment un important dégagement de chaleur lors du massage, et l'apport d'une hydratation importante à la peau.

La composition selon l'invention peut contenir un seul composé lipophile, ou une pluralité de tels composés.

La quantité totale de composé(s) lipophile(s) dans la composition est de préférence supérieure ou égale à 40 % en poids par rapport au poids total de la composition, et préférentiellement comprise entre 40 et 70 % en poids par rapport au poids total de la composition.

Le composé lipophile peut notamment être choisi parmi les triglycérides, de préférence les triglycérides à chaînes moyennes, c'est-à-dire dans lesquels la chaîne hydrocarbonée comporte de 8 à 12 atomes de carbone, et préférentiellement les triglycérides d'acides caprique et/ou caprylique, ou l'un quelconque de leurs mélanges.

Les triglycérides d'acides caprique et/ou caprylique, également nommés triglycérides caprylique/caprique, présentent avantageusement un effet émollient, qui accroit plus encore le degré d'hydratation de la peau, et un effet nutritif. Ils sont en outre issus de ressources renouvelables.

La composition selon l'invention peut contenir un seul agent émulsionnant, ou une pluralité de tels agents.

Ce ou ce(s) agent(s) émulsionnant(s) sont choisis de telle sorte que la composition se présente sous forme d'une microémulsion translucide stable, ayant la texture d'un gel, de préférence à propriétés rhéofluidifiantes, la composition comprenant une phase grasse et une phase aqueuse, formées par exemple, respectivement, d'au moins un composé lipophile tel que des triglycérides caprylique/caprique, et d'au moins un polyol tel que la glycérine et une petite quantité d'eau.

Ainsi, l'agent émulsionnant présente de préférence des propriétés gélifiantes.

L'agent émulsionnant peut contenir au moins, ou être constitué d'au moins, un composé choisi parmi :
- les esters d'acides gras et de polyglycérine ; un tel ester ou un mélange de tels esters étant préférentiellement présent dans la composition selon l'invention dans une quantité totale comprise entre 1 et 15% en poids par rapport au poids total de la composition.

L'agent émulsionnant peut contenir, en mélange :
- un ester de sucre, de préférence un ester de disaccharide, tel qu'un ester de sucrose, de lactose, de maltose, ou l'un quelconque de leurs mélanges ; et préférentiellement un ester de sucrose ; ou encore un ester de monosaccharide, tel qu'un ester de glucose, de fructose, de galactose ; ou un mélange de tels esters ;
- un polyol, notamment de la glycérine ;
- et un composé lipophile choisi parmi les triglycérides, de préférence les triglycérides à chaînes moyennes, et préférentiellement les triglycérides d'acides caprique et/ou caprylique, ou l'un quelconque de leurs mélanges.

L'ester de sucrose peut notamment être du laurate de sucrose, ou tout autre ester du sucrose comprenant une chaîne hydrocarbonée, notamment une chaîne alkyl, de 4 à 30 atomes de carbone, de préférence de 12 à 18 atomes de carbone, cette chaine pouvant être linéaire ou ramifiée, saturée ou insaturée, et même cyclique ou aromatique.

L'ester du sucrose est préférentiellement présent dans la composition selon l'invention dans une quantité totale comprise entre 1 et 10 %, de préférence entre 1 et 5 %, en poids par rapport au poids total de la composition.

La mise en œuvre de glycérine et de triglycérides caprylique/caprique dans l'agent émulsionnant selon l'invention est particulièrement avantageuse dans les modes de réalisation particuliers de la composition selon l'invention décrits ci-avant dans lesquels le composé lipophile est un triglycéride caprylique/caprique et/ou l'agent humectant est la glycérine. Le nombre total de composants dans la composition selon l'invention est alors réduit.

Dans des modes de réalisation particuliers de la composition selon l'invention, l'agent émulsionnant contient en mélange :
- 6,5 % en poids, par rapport au poids total de l'agent émulsionnant, de laurate de sucrose,
- 46 % en poids, par rapport au poids total de l'agent émulsionnant, de glycérine,
- 35 % en poids, par rapport au poids total de l'agent émulsionnant, de triglycérides d'acides caprylique et/ou caprique,
- 13,5 % en poids, par rapport au poids total de l'agent émulsionnant, d'eau.

Un tel agent émulsionnant, qui présente avantageusement des propriétés gélifiantes importantes, et qui permet de former une microémulsion du type huile-dans-eau sous forme de gel à propriétés rhéofluidifiantes, est commercialisé sous la dénomination Sucragel® CF par la société Alfa.

Il s'avère particulièrement avantageux dans le cadre de la présente invention.

Dans d'autres modes de réalisation particuliers de l'invention, l'agent émulsionnant est choisi parmi les esters d'acides gras et de polyglycérine. L'agent émulsionnant est par exemple le polyglycéryl-10 myristate, tel que commercialisé par exemple sous la dénomination SFACE M-1001 par la société Rossow Cosmétiques. Un tel agent émulsionnant permet également de former une microémulsion du type huile-dans-eau sous forme de gel à propriétés rhéofluidifiantes. Il est préférentiellement présent dans la composition selon l'invention dans une quantité totale comprise entre 1 et 15 % en poids, par rapport au poids total de la composition.

La quantité totale d'agent(s) émulsionnant(s) dans la composition est de préférence comprise entre 1 et 30 % en poids par rapport au poids total de la composition, notamment, selon le(s) agent(s) émulsionnant(s) mis en œuvre, par exemple pour le Sucragel® CF, entre 6 et 30 % en poids par rapport au poids total de la composition.

Préférentiellement, la quantité totale d'eau dans la composition est comprise entre 1 et 8 % en poids, par rapport au poids total de la composition.

La composition selon l'invention peut contenir d'autres agents actifs, notamment par voie topique, par exemple choisis parmi les antioxydants, tels que la vitamine E, les bloqueurs de rayonnements ultra violet, les vitamines, les huiles telles que l'huile d'olive, les huiles essentielles, etc., ou un mélange de tels composés.

Elle peut en outre contenir tout additif classique en lui-même pour une composition cosmétique et/ou dermatologique, par exemple un ou plusieurs des ingrédients ci-après : colorant, agent neutralisant, etc.

Préférentiellement, elle est exempte d'agent conservateur. Elle est également préférentiellement dépourvue de parfum, d'agent désinfectant, ou encore d'agent antimicrobien, bactéricide ou bactériostatique, fongistatique ou fongicide, si bien qu'elle respecte l'équilibre cutané.

Elle est préférentiellement exempte de silicone. Elle ne présente notamment pas d'effet occlusif sur la peau.

La composition selon l'invention est en outre préférentiellement dépourvue de beurres ou de cires dans sa phase lipophile.

La composition selon l'invention contient de préférence un faible nombre total d'ingrédients, en particulier un nombre total d'ingrédients inférieur à 12, et de préférence inférieur à 10.

Avantageusement, aucun de ces ingrédients n'est du type à risque d'intolérance, ou du type modifiant la biologie de la peau. Tous sont de préférence des ingrédients physio-compatibles.

Préférentiellement, la grande majorité des ingrédients composant la composition selon l'invention sont naturels ou d'origine naturelle, et peuvent être obtenus par des procédés écologiques / respectueux de l'environnement.

La composition selon l'invention peut être conditionnée dans tout récipient classique en lui-même. Elle peut notamment être conditionnée en tube.

La composition selon l'invention est utilisée pour le traitement, en particulier le remodelage, d'une cicatrice, formée sur la peau d'un mammifère, en particulier d'un humain, par application de ladite composition par voie topique sur ladite cicatrice, et le cas échéant la zone de peau environnante.

Ce traitement vise notamment à améliorer l'aspect esthétique de la cicatrice, ainsi que le confort de l'individu qui la porte, par soulagement des démangeaisons et douleurs associées à cette cicatrice.

Selon l'invention, l'utilisation de la composition comprend les étapes suivantes :
- l'application de la composition par voie topique sur la surface de la cicatrice, dans une quantité efficace pour le traitement de ladite cicatrice,
- le massage de la cicatrice, de préférence par une méthode dite de palper-rouler, de sorte à faire pénétrer la composition dans l'épiderme.

Le massage mis en œuvre provoque notamment le passage de la composition d'une forme de gel à une forme plus fluide, qui pénètre immédiatement dans l'épiderme.

Préférentiellement, la composition est appliquée sur la surface de la cicatrice par des mouvements circulaires, qui provoquent un échauffement de la peau, préparant cette dernière au massage par activation de la microcirculation sanguine dans cette zone. S'ajoute avantageusement à cela l'effet chauffant de l'agent chauffant préférentiellement présent dans la composition.

Le massage à proprement parler, réalisé dans un second temps, notamment par la méthode dite de palper-rouler, exerce quant à lui un effet d'assouplissement de la peau au niveau de la cicatrice, il agit sur les fibres de collagène et contribue de manière importante à un remodelage optimal de la cicatrice.

Ces étapes sont de préférence réalisées une à deux fois par jour.

La composition selon l'invention est de préférence utilisée pendant une période de deux mois à deux ans à partir de la réépidermisation complète de la lésion à l'origine de la cicatrice.

La composition peut être appliquée aussi bien sur la peau du visage que sur celle du corps ou du cuir chevelu. L'individu traité peut aussi bien être un nourrisson, qu'un enfant ou un adulte.

En fin d'application, la composition selon l'invention laisse sur la surface cutanée un film protecteur, donnant l'effet d'une seconde peau respirante, hydratant, non gras et non collant, qui redonne du confort à l'individu traité et réduit l'incitation au grattage.

L'invention s'exprime également dans les termes d'un procédé de traitement, en particulier de remodelage, d'une cicatrice, formée sur la peau d'un mammifère, en particulier d'un humain, par application par voie topique sur ladite cicatrice, et le cas échéant la zone de peau environnante, d'une quantité efficace d'une composition selon l'invention, répondant notamment à l'une ou plusieurs des caractéristiques ci-avant.

Ce procédé peut répondre à l'une ou plusieurs des caractéristiques décrites ci-avant en référence à l'utilisation de la composition selon l'invention.

Ce procédé peut notamment être un procédé de traitement cosmétique de la cicatrice, visant à en améliorer l'aspect esthétique.

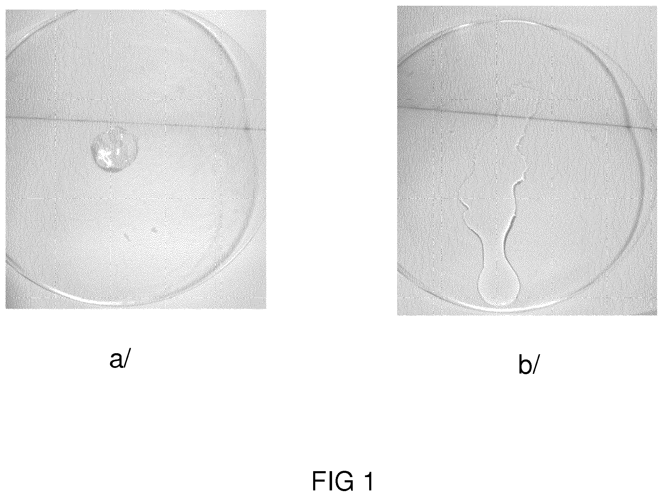

Les caractéristiques et avantages de l'invention apparaîtront plus clairement à la lumière des exemples de mise en œuvre ci-après, fournis à simple titre illustratif et nullement limitatifs de l'invention, avec l'appui de la figure 1, qui illustre la capacité d'écoulement a/ d'une composition selon l'invention, et b/ d'une composition d'huiles de l'art antérieur, 1 g de chacune des compositions ayant été déposés respectivement sur un verre de montre, lequel étant incliné de sorte à provoquer l'écoulement de la composition à sa surface.

### EXEMPLE 1

Il est préparé une composition conforme à la présente invention, à partir des ingrédients indiqués dans le tableau 1 ci-après. Pour chacun de ces ingrédients, la quantité est indiquée en pourcentage en poids, par rapport au poids total de la composition, étant entendu que la quantité d'eau dans la composition est d'au moins 1 %, et ne dépasse pas 10 %, en poids par rapport au poids total de la composition.

**Tableau 1 ― Composition selon l'invention**

| Ingrédient | Quantité (% p/p) |
|---|---|
| Sucragel® CF | 10-30 |
| Triglycérides caprylique / caprique | 40 - 60 |
| Huile d'olive | 1 - 6 |
| Glycérine | 10-40 |
| Hyaluronate de sodium | 0,01 - 1 |
| Extrait de plantule d'avoine | 0,01 - 1 |
| L-alanyl-L-glutamine | 0,01 - 1 |
| Vitamine E | 0,1 - 1,5 |
| Eau | qsp |

La viscosité dynamique de cette composition à l'état initial est mesurée au moyen d'un rhéomètre Lamy Rheology, RM180 Rheomat (mobile de mesure : taille 3 ; vitesse de rotation : 7,61 s⁻¹ ; température : 22 °C).

Cette viscosité dynamique est comprise entre 16000 et 22000 mPa.s.

Pour évaluer la capacité d'écoulement de cette composition, 1 g de composition sont déposés sur un verre de montre, et le verre de montre est incliné de sorte à provoquer l'écoulement de la composition sur sa surface. A titre d'exemple comparatif, la même expérience est réalisée avec le mélange d'huiles commercialisé sous la dénomination Bi-Oil par les laboratoires Omega Pharma. Les résultats obtenus sont montrés sur la figure 1. Comme on peut le voir, alors que la composition Bi-Oil s'écoule le long de la surface du verre (en b/ sur la figure), la composition selon l'invention ne s'écoule pas du tout (en a/ sur la figure), pour la même inclinaison du verre.

Cette composition selon l'invention, se présentant à l'état initial sous forme de gel huileux, est appliquée sur la cicatrice après fermeture totale de la lésion qui en est à l'origine, de préférence à raison d'une à deux fois par jour. A cet effet, il est tout d'abord réalisé des mouvements circulaires, pendant quelques minutes, puis un massage de type palper-rouler.

Lors de la première phase, la forme en gel prépare la peau au massage en activant la microcirculation.

Lors de la deuxième phase, le massage de type palper-rouler assouplit la peau, agit sur les fibres de collagène et d'élastine et contribue ainsi à un remodelage optimal de la peau.

Un tel traitement apaise durablement les sensations de tiraillement et favorise le remodelage de l'épiderme. Après quelques semaines de traitement, les marques cicatricielles et les vergetures sont moins visibles.

A titre d'exemple comparatif, il est préparé une composition similaire, mais dans laquelle, en tant qu'agent émulsionnant, le Sucragel® CF est remplacé par un mélange de cétéaryl glucoside et d'alcool cétéarylique proposé par le document WO 2014/167039 dans son exemple figurant en page 12. Ce mélange de composés ne permet pas de former une microémulsion, ni même une émulsion stable, si bien que la composition obtenue ne s'avère nullement adaptée à un massage prolongé de la surface cutanée.

### EXEMPLE 2

Il est préparé une composition conforme à la présente invention, à partir des ingrédients indiqués dans le tableau 2 ci-après. Pour chacun de ces ingrédients, la quantité est indiquée en pourcentage en poids, par rapport au poids total de la composition, étant entendu que la quantité d'eau dans la composition est d'au moins 1 %, et ne dépasse pas 10 %, en poids par rapport au poids total de la composition.

**Tableau 2 ― Composition selon l'invention**

| Ingrédient | Quantité (% p/p) |
|---|---|
| Polyglycéryl-10 Myristate | 1 - 15 |
| Triglycérides caprylique / caprique | 40 ― 70 |
| Huile d'olive | 1 - 6 |
| Glycérine | 10 - 50 |
| Hyaluronate de sodium | 0,01 - 1 |
| Extrait de plantule d'avoine | 0,01 - 1 |
| L-alanyl-L-glutamine | 0,01 - 1 |
| Vitamine E | 0,1 - 1,5 |
| Eau | qsp |

Cette composition se présente sous forme d'un gel huileux. Sa viscosité à l'état initial, mesurée comme indiqué ci-avant dans l'Exemple 1, à 22 °C, est comprise entre 10000 et 16000 mPa.s.

Cette composition est appliquée sur la cicatrice dans les mêmes conditions que celles décrites ci-avant en référence à l'Exemple 1.

Là encore, le traitement apaise durablement les sensations de tiraillement et favorise le remodelage de l'épiderme. Après quelques semaines de traitement, les marques cicatricielles et les vergetures sont en outre moins visibles.

## Revendications

1. Composition applicable par voie topique, se présentant sous forme d'une microémulsion comprenant une phase lipophile et une phase aqueuse, et sous forme de gel, ladite composition contenant :
- un composé lipophile,
- un agent cicatrisant,
- un agent humectant,
- un agent émulsionnant contenant au moins un composé choisi parmi :
o les esters d'acides gras et de polyglycérine
o et les mélanges d'un ester de sucre, d'un polyol et d'un composé lipophile choisi parmi les triglycérides ou l'un quelconque de leurs mélanges,
- et de l'eau dans une quantité totale comprise entre 1 et 10 % en poids par rapport au poids total de la composition,
pour son utilisation pour le traitement d'une cicatrice, par application de ladite composition par voie topique sur la surface de ladite cicatrice et massage de ladite cicatrice.

2. Composition pour son utilisation selon la revendication 1, selon laquelle ladite composition présente une viscosité dynamique à 22 °C supérieure ou égale à 8 000 mPa.s.

3. Composition pour son utilisation selon l'une quelconque des revendications 1 à 2, selon laquelle ladite composition se présente sous forme de microémulsion de type huile-dans-eau.

4. Composition pour son utilisation selon l'une quelconque des revendications 1 à 3, selon laquelle l'agent cicatrisant contient de l'acide hyaluronique ou un de ses sels.

5. Composition pour son utilisation selon la revendication 4, selon laquelle l'agent cicatrisant contient une association comprenant de l'acide hyaluronique ou un de ses sels, de la L-alanyl-L-glutamine et un extrait d'avoine.

6. Composition pour son utilisation selon l'une quelconque des revendications 1 à 5, selon laquelle la quantité totale d'agent(s) cicatrisant(s) dans la composition est comprise entre 0,01 et 3 % en poids par rapport au poids total de la composition.

7. Composition pour son utilisation selon l'une quelconque des revendications 1 à 6, selon laquelle ladite composition contient un agent, dit agent chauffant, apte à produire de la chaleur lorsqu'il est appliqué sur la surface cutanée.

8. Composition pour son utilisation selon l'une quelconque des revendications 1 à 7, selon laquelle ledit agent humectant est un polyol.

9. Composition pour son utilisation selon la revendication 8, selon laquelle ledit agent humectant est la glycérine, la quantité totale de glycérine étant comprise entre 10 et 50 % en poids par rapport au poids total de la composition

10. Composition pour son utilisation selon l'une quelconque des revendications 1 à 9, selon laquelle le composé lipophile est choisi parmi les triglycérides ou l'un quelconque de leurs mélanges.

11. Composition pour son utilisation selon l'une quelconque des revendications 1 à 10, selon laquelle la quantité totale de composé(s) lipophile(s) dans ladite composition est supérieure ou égale à 40 % en poids par rapport au poids total de la composition.

12. Composition pour son utilisation selon l'une quelconque des revendications 1 à 11, selon laquelle la quantité totale d'agent(s) émulsionnant(s) dans la composition est comprise entre 1 et 30 % en poids par rapport au poids total de la composition.

13. Composition pour son utilisation selon l'une quelconque des revendications 1 à 12, pendant une période de deux mois à deux ans à partir de la réépidermisation complète de la lésion à l'origine de la cicatrice.

## Patentansprüche

1. Zusammensetzung, die auf topischem Weg anwendbar ist, die in Form einer Mikroemulsion vorliegt, umfassend eine lipophile Phase und eine wässrige Phase, und in Gelform, wobei die Zusammensetzung enthält:
- eine lipophile Verbindung,
- ein wundenschließendes Mittel,
- ein Befeuchtungsmittel,
- ein Emulsionsmittel, das mindestens eine Verbindung enthält, die ausgewählt ist aus:
∘ den Fettsäure- und den Polyglycerinestern
∘ und den Gemischen aus einem Zuckerester, einem Polyol und einer lipophilen Verbindung, ausgewählt aus den Triglyceriden oder einem ihrer Gemische,
- und Wasser in einer Gesamtmenge, die zwischen 1 und 10 Gew.-% in Bezug auf das Gesamtgewicht der Zusammensetzung liegt,
für deren Verwendung zur Behandlung eine Narbe durch Auftragen der Zusammensetzung auf topischem Weg auf die Oberfläche der Narbe und Massage der Narbe.

2. Zusammensetzung für deren Verwendung nach Anspruch 1, wobei die Zusammensetzung eine dynamische Viskosität bei 22 °C von über oder gleich 8.000 mPa.s aufweist.

3. Zusammensetzung für deren Verwendung nach einem der Ansprüche 1 bis 2, wobei die Zusammensetzung in Form einer Mikroemulsion vom Typ Öl-in-Wasser vorliegt.

4. Zusammensetzung für deren Verwendung nach einem der Ansprüche 1 bis 3, wobei das wundschließende Mittel Hyaluronsäure oder eines ihrer Salze enthält.

5. Zusammensetzung für deren Verwendung nach Anspruch 4, wobei das wundschließende Mittel eine Kombination enthält, die Hyaluronsäure oder eines ihrer Salze, L-alanyl-L-glutamin und einen Haferextrakt enthält.

6. Zusammensetzung für deren Verwendung nach einem der Ansprüche 1 bis 5, wobei die Gesamtmenge des/r wundenschließenden/er Mittel in der Zusammensetzung zwischen 0,01 und 3 Gew.-% in Bezug auf das Gesamtgewicht der Zusammensetzung liegt.

7. Zusammensetzung für deren Verwendung nach einem der Ansprüche 1 bis 6, wobei die Zusammensetzung eine wärmebildendes Mittel enthält, das imstande ist, Wärme zu produzieren, wenn es auf die Hautoberfläche aufgetragen ist.

8. Zusammensetzung für deren Verwendung nach einem der Ansprüche 1 bis 7, wobei das Befeuchtungsmittel ein Polyol ist.

9. Zusammensetzung für deren Verwendung nach Anspruch 8, wobei das Befeuchtungsmittel Glycerin ist, wobei die Gesamtmenge von Glycerin zwischen 10 und 50 Gew.-% in Bezug auf das Gesamtgewicht der Zusammensetzung liegt.

10. Zusammensetzung für deren Verwendung nach einem der Ansprüche 1 bis 9, wobei die lipophile Verbindung aus den Triglyceriden oder einem ihrer Gemische ausgewählt ist.

11. Zusammensetzung für deren Verwendung nach einem der Ansprüche 1 bis 10, wobei die Gesamtmenge liphophiler Verbindung/en in der Zusammensetzung über oder gleich 40 Gew.-% in Bezug auf das Gesamtgewicht der Zusammensetzung beträgt.

12. Zusammensetzung für deren Verwendung nach einem der Ansprüche 1 bis 11, wobei die Gesamtmenge von Emulgationsmittel/n in der Zusammensetzung zwischen 1 und 30 Gew.-% in Bezug auf das Gesamtgewicht der Zusammensetzung beträgt.

13. Zusammensetzung für deren Verwendung nach einem der Ansprüche 1 bis 12 während eines Zeitraums von zwei Monaten bis zu zwei Jahren ab der vollständigen Neubildung der Epidermis der Verletzung als Ursache der Narbe.

## Claims

1. Topically applicable composition, in the form of a microemulsion comprising a lipophilic phase and an aqueous phase, and in gel form, said composition containing:
- a lipophilic compound,
- a wound-healing agent,
- a humectant,
- an emulsifier containing at least a compound chosen from:
o esters of fatty acids and polyglycerol
o and mixtures of a sugar ester, a polyol and a lipophilic compound chosen from triglycerides or any one of the mixtures thereof,
- and water in a total amount between 1% and 10% by weight relative to the total weight of the composition,
for its use in treating a scar, by topical application of said composition to the surface of said scar and massaging of said scar.

2. Composition for its use according to claim 1, wherein said composition has a dynamic viscosity at 22°C of greater than or equal to 8000 mPa.s.

3. Composition for its use according to any one of claims 1 to 2, wherein said composition is in the form of a microemulsion of oil-in-water type.

4. Composition for its use according to any one of claims 1 to 3, wherein the wound-healing agent contains hyaluronic acid or a salt thereof.

5. Composition for its use according to claim 4, wherein the wound-healing agent contains a combination comprising hyaluronic acid or a salt thereof, L-alanyl-L-glutamine and an oat extract.

6. Composition for its use according to any one of claims 1 to 5, wherein the total amount of wound-healing agent(s) in the composition is between 0.01% and 3% by weight relative to the total weight of the composition.

7. Composition for its use according to any one of claims 1 to 6, wherein said composition contains an agent, called heating agent, capable of producing heat when it is applied to the skin surface.

8. Composition for its use according to any one of claims 1 to 7, wherein said humectant is a polyol.

9. Composition for its use according to claim 8, wherein said humectant is glycerol, the total amount of glycerol being comprised between 10% and 50% by weight relative to the total weight of the composition.

10. Composition for its use according to any one of claims 1 to 9, wherein the lipophilic compound is chosen from triglycerides or any one of the mixtures thereof.

11. Composition for its use according to any one of claims 1 to 10, wherein the total amount of lipophilic compound(s) in said composition is greater than or equal to 40% by weight relative to the total weight of the composition.

12. Composition for its use according to any one of claims 1 to 11, wherein the total amount of emulsifier(s) in the composition is between 1% and 30% by weight relative to the total weight of the composition.

13. Composition for its use according to any one of claims 1 to 12, for a period of two months to two years starting from complete re-epidermization of the lesion responsible for the scar.
